# EUROPEAN PATENT SPECIFICATION

(11) **EP 2 661 180 B1**
(45) Date of publication and mention of the grant of the patent: **28.09.2016**
(21) Application number: 11808740.2
(22) Date of filing: 09.12.2011
(51) Int. Cl.: A23L 33/00, A23L 33/17

(54) **COMPOSITION COMPRISING HEAT LABILE MILK PROTEINS AND PROCESS FOR PREPARING SAME**
ZUSAMMENSETZUNG MIT HITZELABILEN MILCHPROTEINEN UND VERFAHREN ZU IHRER HERSTELLUNG
COMPOSITION CONTENANT DES PROTÉINES DE LAIT THERMOLABILES ET PROCÉDÉ DE PRÉPARATION ASSOCIÉ

(30) Priority: 05.01.2011 US 984660
(43) Date of publication of application: 13.11.2013
(73) Proprietor: MJN U.S. Holdings LLC, Glenview, IL 60026 (US)
(72) Inventor: BANAVARA, Dattatreya, Evansville IN 47714 (US); ALVEY, John, D., Evansville IN 47711 (US); GONZALEZ, Juan, M., Newburgh IN 47630 (US)
(74) Representative: V.O.
(86) International application number: PCT/US2011/064054
(87) International publication number: WO 2012/094098

(56) References cited:
- EP-A1- 2 251 031
- WO-A1-98/06425
- WO-A1-2009/068549
- US-A- 5 952 295
- US-A1- 2003 124 237
- US-A1- 2008 003 329
- US-A1- 2010 284 980

## Description

### TECHNICAL FIELD

This disclosure relates generally to the field of nutritional compositions, such as infant formulas, human milk fortifiers, children's dietary supplements, and the like containing heat labile milk proteins, and processes for preparing such compositions.

### BACKGROUND ART

Several proteins naturally found in milk have useful biological activities. These proteins can be found in whole milk, as well as whey, casein or other milk protein fractions or isolates. For example, the protein lactoferrin, found in milk of humans and non-humans, has a number of different antibacterial and antiviral activities. Other milk proteins, including lactoperoxidase and lactadherin (milk fat globule-EGF factor 8 protein), also have been said to be beneficial in reducing the risk of infections. Accordingly, it has been desirable to attempt to include these biologically active proteins in milk-based dietary compositions for humans, such as infant formulas.

Unfortunately, attempts to include biologically active proteins in milk-based dietary compositions are often frustrated by the fact that the biological activities of certain milk proteins can be lost or significantly diminished under the temperature conditions typically used to provide sanitary milk-based compositions for human consumption. More specifically, many milk proteins are denatured or otherwise inactivated by heat processing methods. For example, lactoferrin and other biologically active milk proteins, such as lactoperoxidase and lactadherin, are unstable to some extent when subjected to pasteurization conditions, such as 72°C for 15 seconds. Other milk proteins subject to denaturing or inactivation under conditions of high heat are lactoferricin and transforming growth factor (TGF-β). Such proteins are particularly vulnerable under harsher processing conditions, such as treatment at 130°C to 145°C.
US 2008/003329 describes nutritional compositions enriched with and comprising select combinations of phospholipids, lactoferrin, gangliosides, and sialic acid, which are maintained at room temperature.
US 5,952,295 describes an enteral, nutritional composition comprising casein rich in TGF-β2 for the treatment or prophylaxis of inflammatory conditions of the gastro-intestinal tract without specifying temperature conditions for preparing the nutritional composition.
US 2010/284980 describes a nutritional composition comprising TGF-β for improving digestibility without specifying temperature conditions for preparing the nutritional composition.
EP 2 251 031 describes a composition comprising lactoferrin that can be used for the treatment or prevention of a delayed development of the enteric nervous system. The document describes sterilization at 80-110°C.
WO 98/06425 describes a pharmaceutical composition comprising lactoferrin and/or lactoferricin for treatment and/or prevention of infections, inflammations and/or tumours.
WO 2009/068549 describes the use of a protein source comprising whey and casein proteins for providing an age-tailored nutrition system to an infant, which may contain lactoferrin. It describes thermally treating the composition at 80-110°C to reduce bacterial load.

Accordingly, it would be beneficial to provide a process for preparing a nutritional composition, such as an infant formula, human milk fortifier, children's dietary supplement, and the like, which has been subjected to high temperature processing conditions but contains a heat labile milk protein that is biologically active.

### DISCLOSURE OF THE INVENTION

Briefly, the present disclosure is directed, in an embodiment, to a method for preparing a composition comprising a fat or lipid source, a protein source and a heat labile milk protein. The method includes: a) providing a first composition comprising a fat or lipid source and a protein source and subjecting the first composition to a temperature of at least about 130°C; b) providing a second composition comprising a heat labile milk protein; and c) combining the first composition with a second composition to form a third composition comprising a fat or lipid source, a protein source and a heat labile milk protein wherein the second composition and the third composition are not subjected to a temperature of greater than about 80°C. In a preferred embodiment, the first and third compositions are nutritional compositions.

In certain embodiments, the first composition contains up to about 7 g/100 kcal of a fat or lipid source, more preferably about 3 g/100 kcal to about 7 g/100 kcal of a fat or lipid source, and up to about 5 g/100 kcal of a protein source, more preferably about 1 g/100 kcal to about 5 g/100 kcal of a protein source.

Preferably, the heat labile milk protein in the second composition is lactoferrin, lactoperoxidase lactoferricin, TGF-β and/or lactadherin, more preferably the heat labile milk protein is lactoferrin. It is especially preferred that the heat labile milk protein is lactoferrin produced by a non-human source.

In certain embodiments, the second composition is a solution, preferably an aqueous solution that includes water that has been treated for food processing, such as by reverse osmosis, ultraviolet (UV) light treatment, irradiation, electric pulse, high heat, etc.. In one embodiment, the second composition has been filtered prior to combining the composition with the first composition. In another embodiment, the heat labile milk protein has been subjected to sterilization when the second composition is combined with the first composition. In yet another embodiment, the method further includes packaging the third composition aseptically. Neither the second composition nor the third composition is subjected to a temperature of greater than about 80°C.

Further described is a method for preparing a composition including steps of combining a first composition, which comprises a fat or lipid source and a protein source, that has been subjected to a temperature of at least about 130°C with a second composition comprising a heat labile milk protein which has not been subjected to a temperature of about 80°C or higher to form a third composition comprising a fat or lipid source, a protein source and a heat labile milk protein; and packaging the third composition aseptically.

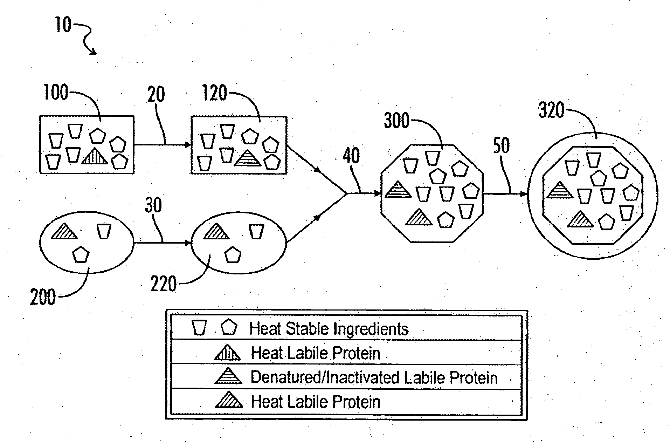

Numerous other objects, features and advantages of the present disclosure will be readily apparent to those skilled in the art upon a reading of the following description when taken in conjunction with the accompanying drawing figure.

### BRIEF DESCRIPTION OF THE DRAWING

The appended figure is a flowchart exemplifying one embodiment of a method in accordance with the present disclosure.

### BEST MODE FOR CARRYING OUT THE INVENTION

In one embodiment, the disclosure is directed to a method for preparing a composition comprising a fat or lipid source, a protein source, and a heat labile milk protein comprising the steps of: a) subjecting a first composition comprising a fat or lipid source and a protein source to a temperature of at least about 130°C; b) providing a second composition comprising a heat labile milk protein; and c) combining the first composition with the second composition to form a third composition comprising a fat or lipid source, a protein source, and a heat labile milk protein wherein the second composition and the third composition have not been subjected to a temperature of about 80°C or higher.

Suitable fat or lipid sources useful for inclusion in the first composition may be any known or used in the art, including but not limited to, animal sources, e.g., milk fat, butter, butter fat, egg yolk lipid; marine sources, such as fish oils, marine oils, single cell oils; vegetable and plant oils, such as corn oil, canola oil, sunflower oil, soybean oil, palmolein, coconut oil, high oleic sunflower oil, evening primrose oil, rapeseed oil, olive oil, flaxseed (linseed) oil, cottonseed oil, high oleic safflower oil, palm stearin, palm kernel oil, wheat germ oil; medium chain triglyceride oils and emulsions and esters of fatty acids; and any combinations thereof.

In certain embodiments, the protein source included in the first composition comprises bovine milk proteins. Bovine milk protein sources useful for inclusion in the first composition include, but are not limited to, milk protein powders, milk protein concentrates, milk protein isolates, nonfat milk solids, nonfat milk, nonfat dry milk, whey protein, whey protein isolates, whey protein concentrates, sweet whey, acid whey, casein, acid casein, caseinate (*e.g.* sodium caseinate, sodium calcium caseinate, calcium caseinate) and any combinations thereof.

In one embodiment, the proteins are provided as intact proteins. In other embodiments, the proteins are provided as a combination of both intact proteins and partially hydrolyzed proteins, with a degree of hydrolysis of between about 4% and 10%. In still other embodiments the proteins are extensively hydrolyzed, with a degree of hydrolysis of greater than 15%, even greater than 50%, and even as high as 90% or higher. In yet another embodiment, the protein source may be supplemented with glutamine-containing peptides.

In a particular embodiment of the disclosure, the protein source comprises whey and casein proteins and the ratio of whey to casein proteins ratio is similar to that found in human breast milk. For example, in certain embodiments, the weight ratio of whey to casein proteins is from about 20% whey:80% casein to about 80% whey:20% casein.

In certain embodiments, the first composition and/or the third composition can be classified as an infant formula. The term "infant formula" applies to a composition in liquid or powdered form that satisfies the nutrient requirements of an infant by being a substitute for human milk. In the United States, the content of an infant formula is dictated by the federal regulations set forth at 21 C.F.R. §§100, 106 and 107. These regulations define macronutrient, vitamin, mineral, and other ingredient levels in an effort to simulate the nutritional and other properties of human breast milk. In a separate embodiment, the first composition and/or the third composition may be a human milk fortifier, meaning it is a composition which is added to human milk in order to enhance the nutritional value of human milk. As a human milk fortifier, the third composition may be in powder or liquid form. In yet another embodiment, the disclosed first composition and/or the third composition may be a children's nutritional composition.

The first composition may be subjected to a temperature of 130°C using equipment and processes familiar to the skilled artisan. Preferably, the first composition is subjected to a temperature of from about 130°C to about 150°C for a time period of at least about 1 second. More preferably, the first composition is subjected to a temperature of from about 130°C to about 150°C for a time period of from about 3 seconds to about 30 seconds.

The first composition comprising a fat or lipid source and a protein source is combined with a second composition comprising a heat labile milk protein.

As used herein, a "heat labile milk protein" is a protein 1) that is either naturally found in milk of at least one type of mammal (i.e., a protein that has an amino acid sequence that is substantially identical to a protein naturally found in milk of at least one type of mammal) or a protein that is an amino acid variant of a protein naturally found in milk of at least one type of mammal; and 2) whose biological activity is lost or diminished when subjected to an elevated temperature, such as greater than 80°C, or, in some cases, temperatures of at least about 130°C. Preferably, the heat labile milk protein is naturally found in milk of at least one type of mammal. Such proteins may be, for example, isolated from milk of at least one type of mammal or produced by a genetically modified organism. In another embodiment, the heat labile milk protein is an amino acid variant of a protein naturally found in milk of at least one type of mammal that is prepared by, for example, removing, substituting, or adding one or more amino acids from or to the amino acid sequence of a protein naturally found in milk of at least one type of mammal. Heat labile milk proteins for use in the present disclosure, include, but are not limited to, lactoferrin, lactadherin, lactoperoxidase, lactoferricin, TGF-β, lysozyme and immunoglobulins. Preferably, the heat labile milk protein is lactoferrin, lactadherin and/or lactoperoxidase. It is especially preferred that the heat labile milk protein is lactoferrin.

Lactoferrins are single chain polypeptides of about 80 kD containing 1 - 4 glycans, depending on the species. The 3-D structures of lactoferrin of different species are very similar, but not identical. Each lactoferrin comprises two homologous lobes, called the N- and C-lobes, referring to the N-terminal and C-terminal part of the molecule, respectively. Each lobe further consists of two sub-lobes or domains, which form a cleft where the ferric ion (Fe³⁺) is tightly bound in synergistic cooperation with a (bi)carbonate anion. These domains are called N1, N2, C1 and C2, respectively. The N-terminus of lactoferrin has strong cationic peptide regions that are responsible for a number of important binding characteristics. Lactoferrin has a very high isoelectric point (∼pI 9) and its cationic nature plays a major role in its ability to defend against bacterial, viral, and fungal pathogens. There are several clusters of cationic amino acids residues within the N-terminal region of lactoferrin mediating the biological activities of lactoferrin against a wide range of microorganisms. For instance, the N-terminal residues 1-47 of human lactoferrin (1-48 of bovine lactoferrin) are critical to the iron-independent biological activities of lactoferrin. In human lactoferrin, residues 2 to 5 (RRRR) and 28 to 31 (RKVR) are arginine-rich cationic domains in the N-terminus especially critical to the antimicrobial activities of lactoferrin. A similar region in the N-terminus is found in bovine lactoferrin (residues 17 to 42; FKCRRWQWRMKKLGAPSITCVRRAFA).

As described in *"*Perspectives on Interactions Between Lactoferrin and Bacteria" which appeared in the publication BIOCHEMISTRY AND CELL BIOLOGY, pp 275-281 (2006), lactoferrins from different host species may vary in their amino acid sequences though commonly possess a relatively high isoelectric point with positively charged amino acids at the end terminal region of the internal lobe. Suitable lactoferrins for use in the present disclosure include those having at least 48% homology with the amino acid sequence AVGEQELRKCNQWSGL at the HLf (349-364) fragment. In some embodiments, the lactoferrin has at least 65% homology with the amino acid sequence AVGEQELRKCNQWSGL at the HLf (349-364) fragment, and, in embodiments, at least 75% homology. For example, non-human lactoferrins for use in the present disclosure include, without limitation, bovine lactoferrin, porcine lactoferrin, equine lactoferrin, buffalo lactoferrin, goat lactoferrin, murine lactoferrin and camel lactoferrin.

In a preferred embodiment, the lactoferrin is lactoferrin produced by a non-human source. As used herein, "lactoferrin produced by a non-human source" means lactoferrin which is produced by or obtained from a source other than human breast milk. For example, in certain embodiments, the lactoferrin is human lactoferrin produced by a genetically modified organism and/or non-human lactoferrin. The term "organism", as used herein, refers to any contiguous living system, such as animal, plant, fungus or micro-organism. The term "non-human lactoferrin", as used herein, refers to lactoferrin having an amino acid sequence that is different than the amino acid sequence of human lactoferrin. It is also preferred that the lactoferrin is not hydrolyzed. In an embodiment, the lactoferrin is bovine lactoferrin. In certain embodiments, the lactoferrin is provided as an isolate and in others as a component of an enriched whey fraction.

In U.S. Patent No. 4,791,193 Okonogi et al. discloses a process for producing bovine lactoferrin in high purity. Generally, the process as disclosed includes three steps. Raw milk material is first contacted with a weakly acidic cationic exchanger to absorb lactoferrin followed by the second step where washing takes place to remove nonabsorbed substances. A desorbing step follows where lactoferrin is removed to produce purified bovine lactoferrin. Other methods may include steps as described in U.S. Patent Nos. 7,368,141, 5,849,885, 5,919,913 and 5,861,491.

In certain embodiments, the heat labile milk protein has been subjected to sterilization (without the application of temperatures in excess of 80°C) prior to combination with the first composition. In one embodiment, the second composition has been filtered through one or more filters, preferably a filter that itself has been subject to sterilization, prior to combining with the first composition.

It will be appreciated that, in certain embodiments, the first composition, which is subjected to a temperature of at least about 130°C, may itself contain a heat labile milk protein. This might be the case if, for example, the biological activities of this heat labile milk protein are not critical. Thus, in certain embodiments, a first composition, which includes a fat or lipid source, a protein source and a heat labile milk protein, is subjected to a temperature of at least about 130°C and then combined with a second composition that includes a heat labile milk protein, preferably a different heat labile milk protein than that present in the first composition, wherein the second composition is not subjected to a temperature of greater than about 80°C.

The step of combining the first and second compositions can be accomplished by processes familiar to the skilled artisan. More particularly, in certain embodiments, the combination of the first and second compositions may be accomplished by aseptic dosing, and can be performed in either a continuous process or a batch process. For example, in one embodiment, the second composition is an aqueous solution, more preferably an aqueous solution that comprises water that has been treated for food processing, such as by reverse osmosis. The solution is then added to the first composition, which is preferably in liquid form. In a particularly preferred embodiment, the first composition is in the form of a liquid composition and the second composition is in the form of a solution that has been subjected to sterilization and the first composition is dosed with a stream of the second composition.

In another preferred embodiment, a process of preparing a composition comprising a heat labile milk protein includes: a) subjecting a liquid nutritional composition comprising a fat or lipid source and a protein source to a temperature of at least about 130°C; b) preparing a solution including a heat labile milk protein; c) subjecting the solution to sterilization; and d) combining the liquid nutritional composition with the solution.

In yet another preferred embodiment, the third composition including lactoferrin is prepared by a process including: a) subjecting a liquid nutritional composition comprising a fat or lipid source and a protein source to a temperature of at least about 130°C to form a first composition; b) preparing a solution including lactoferrin at a lactoferrin concentration of at least 1%; in some embodiments, the lactoferrin concentration is from about 1% to about 30%, and in other embodiments the lactoferrin concentration is from about 1% to about 20%, to form a second composition; c) subjecting the second composition to sterilization at a temperature of no greater than 80°C; and d) combining the first composition with the second composition to form a third composition. In one embodiment, the method includes preparing a solution comprising 1-20% lactoferrin and water that has been treated for food processing, such as by reverse osmosis. It is also preferred the step of subjecting the second composition to sterilization includes filtering the second composition through one or more filters (preferably a filter that itself has been subjected to sterilization) at a temperature of below about 60°C, and preferably from about 4°C to about 60°C. In one embodiment, the pH of the second composition, when a solution, is from about 2 to 7. In some embodiments, the step of combining the liquid nutritional composition (i.e., the first composition) with the lactoferrin solution (i.e., the second composition) to form the third composition includes dosing the liquid nutritional composition with a stream of the lactoferrin solution.

In an embodiment, the amount of the second composition including lactoferrin that is combined with the first composition is selected so that lactoferrin is present in the third composition in an amount of from about 0.1 g/L to about g/L. In another embodiment, the amount of the second composition including lactoferrin that is combined with the first composition is selected so that lactoferrin is present in the third composition in an amount of at least about 10 mg/100 kCal, especially when the nutritional composition is intended for use by children. In certain embodiments, the upper limit of lactoferrin in the third composition is about 300 mg/100 kCal. In another embodiment, where the third composition is an infant formula, lactoferrin is present in the third composition in an amount of from about 70 mg to about 220 mg/100 kCal; in yet another embodiment, lactoferrin is present in the third composition in an amount of about 90 mg to about 190 mg/100 kCal.

After combining the first and second compositions, additional processing steps can be performed upon the third composition, provided any such additional processing steps do not result in inactivation or denaturing of any intact heat labile proteins in the third composition. For example, in a preferred embodiment, the third composition is packaged aseptically, either immediately after the step of combining the first and second compositions or after one or more additional steps. In another embodiment, either immediately after the step of combining the first and second compositions or after one or more additional steps, the third composition is combined with packaging that has been sterilized and sealed under sterilized conditions, such as at a temperature of between about 4°C and about 30°C. In still another embodiment, after combining the first and second compositions, the third composition is reduced to powder form by, for example, freeze drying. The powder then may be reconstituted in liquid form by, for example, adding the powder to milk or water, prior to administration to a human.

The third composition that is produced by the processes disclosed herein may provide minimal, partial, or total nutritional support. The composition may be nutritional supplement or meal replacement. In some embodiments, the composition may be administered in conjunction with a food or another nutritional composition. In this embodiment, the composition can either be intermixed with the food or other nutritional composition prior to ingestion by the subject or can be administered to the subject either before or after ingestion of a food or nutritional composition. In certain embodiments, the third composition is administered to an infant or a child. A "child" and "children" are defined as humans over the age of months to about 12 years old. The term "infant" is generally defined as a human from birth to 12 months of age. In certain embodiments, the composition may be administered to preterm infants receiving infant formula, breast milk, a human milk fortifier, or combinations thereof. A "preterm infant" is an infant born after less than 37 weeks gestation, while a "full term infant" means an infant born after at least 37 weeks gestation.

The third composition may, but need not, an infant formula and may be nutritionally complete. The skilled artisan will recognize "nutritionally complete" to vary depending on a number of factors including, but not limited to, age, clinical condition, and dietary intake of the subject to whom the term is being applied. In general, "nutritionally complete" means that the composition of the present disclosure provides adequate amounts of all carbohydrates, lipids, essential fatty acids, proteins, essential amino acids, conditionally essential amino acids, vitamins, minerals, and energy required for normal growth. As applied to nutrients, the term "essential" refers to any nutrient which cannot be synthesized by the body in amounts sufficient for normal growth and to maintain health and which therefore must be supplied by the diet. The term "conditionally essential" as applied to nutrients means that the nutrient must be supplied by the diet under conditions when adequate amounts of the precursor compound is unavailable to the body for endogenous synthesis to occur.

The composition which is "nutritionally complete" for the preterm infant will, by definition, provide qualitatively and quantitatively adequate amounts of all carbohydrates, lipids, essential fatty acids, proteins, essential amino acids, conditionally essential amino acids, vitamins, minerals, and energy required for growth of the preterm infant. The composition which is "nutritionally complete" for the full term infant will, by definition, provide qualitatively and quantitatively adequate amounts of all carbohydrates, lipids, essential fatty acids, proteins, essential amino acids, conditionally essential amino acids, vitamins, minerals, and energy required for growth of the full term infant. The composition which is "nutritionally complete" for a child will, by definition, provide qualitatively and quantitatively adequate amounts of all carbohydrates, lipids, essential fatty acids, proteins, essential amino acids, conditionally essential amino acids, vitamins, minerals, and energy required for growth of a child.

The third composition may be provided in any form known in the art, including a powder, a gel, a suspension, a paste, a solid, a liquid, a liquid concentrate, or a ready-to-use product. In one preferred embodiment, the third composition is an infant formula, especially an infant formula adapted for use as sole source nutrition for an infant.

In the preferred embodiments, the third composition may be administered enterally. As used herein, "enteral" means through or within the gastrointestinal, or digestive, tract, and "enteral administration" includes oral feeding, intragastric feeding, transpyloric administration, or any other introduction into the digestive tract.

Preferably, the third composition prepared according to the present disclosure include one or more prebiotics, one or more probiotics, and/or one or more source of long chain polyunsaturated fatty acids. As used herein, the term "probiotic" means a microorganism with low or no pathogenicity that exerts beneficial effects on the health of the host and the term "prebiotic" means a non-digestible food ingredient that beneficially affects the host by selectively stimulating the growth and/or activity of one or a limited number of bacteria in the colon that can improve the health of the host.

Including one or more prebiotics, one or more probiotics, and/or one or more source of long chain polyunsaturated fatty acids (LCPUFAs) in the compositions according to the present disclosure can be accomplished by several ways. For example, in one embodiment, one or more of these components, such as the prebiotics and/or the long chain polyunsaturated fatty acids, are included in the first composition prior to subjecting the composition to a temperature of at least about 130°C. In yet another embodiment, one or more of these components, such as the prebiotics and/or the long chain polyunsaturated fatty acids are present in the first composition after to subjecting the composition to a temperature of at least about 130°C but prior to combining with the second composition. In other embodiments, any probiotics included are added to the third composition, such as during aseptic processing. Preferably, if it is desirable to include a prebiotic, a probiotic, and/or a source of long chain polyunsaturated fatty acids that loses its activity upon being subjected to such temperature conditions, it is either included in the third composition or the second composition, or added after the first composition has been subjected to a temperature of at least about 130°C.

As mentioned, in one embodiment, one or more probiotics may be included in accordance with the present disclosure. Any probiotic known in the art may be acceptable in this embodiment provided it achieves the intended result. In a particular embodiment, the probiotic may be selected from *Lactobacillus* species, *Lactobacillus rhamnosus* GG, *Bifidobacterium* species, *Bifidobacterium brevis, Bifidobacterium longum,* and *Bifidobacterium animalis subsp. lactis* BB-12.

If included, the amount of the probiotic in the third composition may vary from about 10⁴ to about 10¹⁰ colony forming units (cfu) per kg body weight per day. In another embodiment, the amount of the probiotic may vary from about 10⁶ to about 10⁹ cfu per kg body weight per day. In yet another embodiment, the amount of the probiotic may be at least about 10⁶ cfu per kg body weight per day. Moreover, the disclosed composition may also include probiotic-conditioned media components.

In an embodiment, one or more of the probiotic(s) is viable. In another embodiment, one or more of the probiotic(s) is non-viable. As used herein, the term "viable" refers to live microorganisms. The term "non-viable" or "non-viable probiotic" means non-living probiotic microorganisms, their cellular components and metabolites thereof. Such non-viable probiotics may have been heat-killed or otherwise inactivated but retain the ability to favorably influence the health of the host. The probiotics useful in the present disclosure may be naturally-occurring, synthetic or developed through the genetic manipulation of organisms, whether such new source is now known or later developed. If a viable probiotic is used, preferably, the probiotic is either included in the second composition or in the third composition.

One or more prebiotics may also be used composition in accordance with the present disclosure. Such prebiotics may be naturally-occurring, synthetic, or developed through the genetic manipulation of organisms and/or plants, whether such new source is now known or developed later. In certain embodiments, the prebiotic included in the compositions of the present disclosure include those taught by U.S. Patent No. 7,572,474. . Prebiotics useful in the present disclosure may include oligosaccharide polysaccharides, and other prebiotics that contain fructose, xylose, soya, galactose, glucose and mannose. More specifically, prebiotics useful in the present disclosure may include lactulose, lactosucrose, raffinose, gluco-oligosaccharide, inulin, polydextrose, polydextrose powder, galactooligosaccharide, fructo-oligosaccharide, isomalto-oligosaccharide, soybean oligosaccharides, lactosucrose, xylooligosacchairde, chito-oligosaccharide, manno-oligosaccharide, aribinooligosaccharide, siallyl-oligosaccharide, fuco-oligosaccharide, and gentiooligosaccharides. Preferably, the prebiotic is polydextrose and/or galactooligosaccaharide. Optionally, in addition to polydextrose and/or galactooligosaccaharide, one or more additional prebiotics are used in accordance with the present disclosure.

In one embodiment, the prebiotics are included such that the total amount of prebiotics present in the third composition is from about 0.1 g/100 kcal to about 1 g/100 kcal. More preferably, the total amount of prebiotics present in the third composition may be from about 0.3 g/100 kcal to about 0.7 g/100 kcal. At least 20% of the prebiotics should comprise galactooligosaccharide (GOS) and/or polydextrose (PDX).

If polydextrose is used, the amount of polydextrose in the third composition may, in an embodiment, be within the range of from about 0.1 g/100 kcal to about 1 g/100 kcal. In another embodiment, the amount of polydextrose in the third composition is within the range of from about 0.2 g/100 kcal to about 0.6 g/100 kcal.

If galactooligosaccharide is used, the amount of galactooligosaccharide in the third composition may, in an embodiment, be from about 0.1 g/100 kcal to about 1 g/100 kcal. In another embodiment, the amount of galactooligosaccharide in the third composition may be from about 0.2 g/100 kcal to about 0.5 g/100 kcal. In certain embodiments, the ratio of polydextrose to galactooligosaccharide in the third composition is between about 9:1 and about 1:9.

One or more sources of long chain polyunsaturated fatty acids may also be used in accordance with the present disclosure. Preferably, the source of LCPUFAs comprise docosahexanoic acid (DHA). Other suitable LCPUFAs include, but are not limited to, α-linoleic acid, γ-linoleic acid, linoleic acid, linolenic acid, eicosapentanoic acid (EPA) and arachidonic acid (ARA).

In one embodiment, the first composition is supplemented with both DHA and ARA. In this embodiment, the weight ratio of ARA:DHA may be from about 1:3 to about 9:1. In one embodiment of the present disclosure, the weight ratio of ARA:DHA is from about 1:2 to about 4:1.

The amount of long chain polyunsaturated fatty acids in the third composition may vary from about 5 mg/100 kcal to about 100 mg/100 kcal, more preferably from about 10 mg/100 kcal to about 50 mg/100 kcal.

The composition may be supplemented with oils containing DHA and ARA using standard techniques known in the art. For example, DHA and ARA may be added to the composition by replacing an equivalent amount of an oil, such as high oleic sunflower oil, normally present in the composition. As another example, the oils containing DHA and ARA may be added to the composition by replacing an equivalent amount of the rest of the overall fat blend normally present in the composition without DHA and ARA.

If utilized, the source of DHA and ARA may be any source known in the art such as marine oil, fish oil, single cell oil, egg yolk lipid, and brain lipid. In some embodiments, the DHA and ARA are sourced from the single cell Martek oil, DHASCO® and ARASCO®, respectively, or variations thereof. The DHA and ARA can be in natural form, provided that the remainder of the LCPUFA source does not result in any substantial deleterious effect on the subject. Alternatively, the DHA and ARA can be used in refined form.

In an embodiment of the present disclosure, sources of DHA and ARA are single cell oils as taught in U.S. Pat. Nos. 5,374,567; 5,550,156; and 5,397,591 However, the present disclosure is not limited to only such oils.

In a particular embodiment, TGF-ß is one of the heat labile proteins present in accordance with the disclosure. TGF-ß may be present in the protein sources used herein in its inactive form. It is then activated in the human gut by enzymes, extremes of pH, and/or tearing. In a particular embodiment, the composition of the disclosure enhances the bioavailability or bioactivity of TGF-ß in the human gut. This may include enhancing the signaling of TGF-ß in the human body. In an embodiment, the composition of the disclosure may enhance the bioactivity of TGF-ß in the human gut by at least about 5%, more advantageously by at least about 15%, or even at least about 25% or higher, up to about 65%.

In a particular embodiment of the disclosure, the third composition includes from about 0.0150 (pg/µg) ppm to about 0.1000 (pg/µg) ppm of TGF-ß. In another embodiment, the level of TGF-ß in the third composition is from about 0.0225 (pg/µg) ppm to about 0.0750 (pg/µg) ppm.

In a particular embodiment of the disclosure, the level of TGF-ß in the disclosed third composition is from about 500 pg/mL to about 10,000 pg/mL composition, more preferably from about 3000 pg/mL to about 8000 pg/mL.

In one embodiment, the ratio of TGF-ß1: TGF-ß2 in the disclosed third composition is in the range of about 1:1 to about 1:20, or, more particularly, in the range of about 1:5 to about 1:15.

In some embodiments, the bioactivity of TGF-ß in a composition is enhanced by the addition of a bioactive whey fraction. Any bioactive whey fraction known in the art may be used in this embodiment provided it achieves the intended result. In an embodiment, this bioactive whey fraction may be a whey protein concentrate. In a particular embodiment, the whey protein concentrate may be Salibra^{®} 800, available from Glanbia Nutritionals. In a particular embodiment, the Salibra^{®} 800 whey protein concentrate is 2.5% acidified. In another embodiment, the Salibra^{®} 800 whey protein concentrate is 5% acidified. In yet another embodiment, the Salibra^{®} 800 whey protein concentrate is 2% acidified. In a further embodiment, the Salibra^{®} 800 whey protein concentrate is 3% acidified.

In another embodiment, the whey protein concentrate may be Nutri Whey 800, available from DMV International. In yet another embodiment, the whey protein concentrate may be Salibra-850, available from Glanbia Nutritionals. In still another embodiment, the whey protein concentrate may be Prolacta Lacatalis WPI90, available from Lactilus Industrie U.S.A., Inc. In a further embodiment, the whey protein concentrate may be supplied by MG Nutritionals.

### EXAMPLES

The following examples are provided to illustrate embodiments of the nutritional composition of the present disclosure but should not be interpreted as any limitation thereon. Other embodiments within the scope of the claims herein will be apparent to one skilled in the art from the consideration of the specification or practice of the nutritional composition or methods disclosed herein.

### EXAMPLE 1

This example illustrates one embodiment of ingredients that can be used to prepare the nutritional product according to the present disclosure.

| | |
|---|---|
| water | 872 ml |
| lactose | 65.6 mg |
| vegetable oil blend | 353.0 mg |
| nonfat milk | 34.0 mg |
| whey protein | 8.5 mg |
| galactooligosaccharide | 4.7 mg |
| casein | 3.5 mg |
| polydextrose | 2.4 mg |
| lactoferrin solution (10%) | 1.0 mg |
| DHA and ARA oil blend | 0.94 mg |
| mono- and di-glycerides | 0.7 mg |
| calcium carbonate | 0.44 mg |
| calcium phosphate | 0.4 mg |
| potassium citrate | 0.4 mg |
| potassium chloride | 0.4 mg |
| lecithin | 0.4 mg |
| sodium chloride | 0.3 mg |
| potassium phosphate | 0.3 mg |
| choline chloride | 0.2 mg |
| magnesium oxide | 0.08 mg |
| calcium hydroxide | 0.08 mg |
| ferrous suflate | 0.07 mg |
| vitamins | 0.03 mg |
| minerals | 0.03 mg |

### EXAMPLE 2

This example illustrates another embodiment of ingredients that can be used to prepare the nutritional product according to the present disclosure.

| | |
|---|---|
| water | 686 ml |
| whey | 215 mg |
| nonfat milk | 67 mg |
| vegetable oil blend | 33 mg |
| lactose | 17 mg |
| galactooligosaccharide | 4.7 mg |
| polydextrose | 2.4 mg |
| lactoferrin solution (10%) | 1.0 mg |
| DHA and ARA oil blend | 0.9 mg |
| mono- and di-glycerides | 0.7 mg |
| calcium carbonate | 0.44 mg |
| calcium phosphate | 0.4 mg |
| potassium citrate | 0.4 mg |
| potassium chloride | 0.4 mg |
| lecithin | 0.4 mg |
| potassium phosphate | 0.3 mg |
| carrageenan | 0.3 mg |
| sodium citrate | 0.2 mg |
| choline chloride | 0.2 mg |
| magnesium oxide | 0.08 mg |
| calcium chloride | 0.08 mg |
| ferrous suflate | 0.07 mg |
| vitamins | 0.03 mg |
| minerals | 0.03 mg |

A nutritional composition containing the above-mentioned components from Examples 1 and 2, except lactoferrin, is prepared in liquid form and is subjected to a temperature of from about 135°C to about 145°C for a time period of from about 3 seconds to about 30 seconds. A 1-30% solution of lactoferrin is prepared in reverse osmosis water and filtered with sterilized filters to create a sterilized solution of lactoferrin. The liquid nutritional composition is combined with the solution of lactoferrin by dosing the liquid nutritional composition with a stream of the lactoferrin solution. The resulting composition is packaged aseptically.

Referring now to the drawing figure, a flowchart for one embodiment of a method in accordance with the present disclosure is denoted by the reference numeral 10. In the method, a nutritional composition 100 is prepared. Nutritional composition 100 may include various heat stable ingredients, as well as one or more heat labile proteins. In processing step 20, nutritional composition 100 is exposed to a temperature of at least 130°C, to form first composition 120, which is sterile; any heat labile proteins in nutritional composition 100 may be inactivated or denatured in first composition 120.

Continuing with the method shown in flowchart 10, a solution 200 containing one or more heat labile proteins is prepared, and subjected to processing step 30, which may include filtration but does not include exposing solution 200 to a temperature of greater than 80°C, to form second composition 220, which is sterile; the heat labile proteins in second composition 220 are not denatured or inactivated.

First composition 120 and second composition 220 are then combined in processing step 40, to form third composition 300 containing the intact heat labile proteins from second composition 220. Third composition 300 is then subjected to aseptic processing and packing in processing step 50, to provide a sterile packaged composition 320.

The discussion of the references herein is intended merely to summar the assertions made by their authors and no admission is made that any reference constitutes prior art.

Although preferred embodiments of the disclosure have been described using specific terms, devices, and methods, such description is for illustrative purposes only. The words used are words of description rather than of limitation. In addition, it should be understood that aspects of the various embodiments may be interchanged both in whole or in part. For example, while methods for the production of a commercially sterile liquid nutritional supplement made according to those methods have been exemplified, other uses are contemplated.

## Claims

1. A method for preparing a composition comprising a fat or lipid source, a protein source and a heat labile milk protein, comprising the steps of:
a) providing a first composition comprising a fat or lipid source and a protein source and subjecting the first composition to a temperature of at least about 130°C;
b) providing a second composition comprising a heat labile milk protein; and
c) combining the first composition with the second composition comprising a heat labile milk protein to form a third composition comprising a fat or lipid source, a protein source and a heat labile milk protein, wherein the second composition and the third composition are not subjected to a temperature of greater than about 80°C.

2. The method according to claim 1, wherein the second composition is an aqueous solution comprising water that has been treated for food processing.

3. The method of Claim 2, wherein the water has been treated by reverse osmosis.

4. The method according to claim 1, wherein the second composition has been filtered through one or more filters prior to combining the second composition with the first composition.

5. The method according to claim 1, further comprising the step of packaging the third composition aseptically.

6. The method according to claim 1, wherein the heat labile milk protein is selected from the group consisting of lactoferrin, lactoferricin, TGF-β, lactoperoxidase, lactadherin, and combinations thereof.

7. The method of claim 6, where lactoferrin is present in the third composition at a level of at least 10 mg/100 kCal.

8. The method of Claim 4, wherein lactoferrin is present in the third composition at a level of from 70 mg/100 kCal to 220 mg/100 kCal.

9. The method according to claim 1, wherein the fat or lipid source is present in the first composition at a level of about 3 g/100 kcal to about 7 g/100 kcal.

10. The method according to claim 1, wherein the protein source is present in the first composition at a level of about 1 g/100 kcal to about 5 g/100 kcal.

11. A composition comprising a fat or lipid source, a protein source, and a heat labile milk protein prepared by the process of any one of the claims 1, 6, 9, or 10.

12. The composition according to claim 11, wherein the process is according to claim 1, and wherein the heat labile milk protein has been subjected to sterilization when the second composition is combined with the first composition.

13. The composition according to claim 11, wherein the process is according to claim 6, and wherein the heat labile milk protein is lactoferrin, preferably selected from the group consisting of non-human lactoferrin, human lactoferrin produced by a genetically modified organism, and combinations thereof.

14. The composition according to claim 11, wherein the process is according to claim 1, and wherein the third composition is an infant formula.

## Patentansprüche

1. Verfahren zur Herstellung einer Zusammensetzung, umfassend eine Fett- oder Lipidquelle, eine Proteinquelle und ein hitzelabiles Milchprotein, umfassend die Schritte von:
a) Bereitstellen einer ersten Zusammensetzung, umfassend eine Fett- oder Lipidquelle und eine Proteinquelle und Aussetzen der ersten Zusammensetzung einer Temperatur von mindestens ungefähr 130°C;
b) Bereitstellen einer zweiten Zusammensetzung, umfassend ein hitzelabiles Milchprotein; und
c) Kombinieren der ersten Zusammensetzung mit der zweiten Zusammensetzung, umfassend ein hitzelabiles Milchprotein, um eine dritte Zusammensetzung, umfassend eine Fett- oder Lipidquelle, eine Proteinquelle und ein hitzelabiles Milchprotein, zu bilden, wobei die zweite Zusammensetzung und die dritte Zusammensetzung nicht einer Temperatur von mehr als ungefähr 80°C ausgesetzt werden.

2. Verfahren nach Anspruch 1, wobei die zweite Zusammensetzung eine wässrige Lösung ist, umfassend Wasser, das für Lebensmittelverarbeitung behandelt wurde.

3. Verfahren nach Anspruch 2, wobei das Wasser durch Umkehrosmose behandelt wurde.

4. Verfahren nach Anspruch 1, wobei die zweite Zusammensetzung durch einen oder mehrere Filter gefiltert wurde, bevor die zweite Zusammensetzung mit der ersten Zusammensetzung kombiniert wurde.

5. Verfahren nach Anspruch 1, ferner umfassend den Schritt vom aseptischen Verpacken der dritten Zusammensetzung.

6. Verfahren nach Anspruch 1, wobei das hitzelabile Milchprotein ausgewählt ist aus der Gruppe bestehend aus Lactoferrin, Lactoferricin, TGF-ß, Lactoperoxidase, Lactadherin und Kombinationen davon.

7. Verfahren nach Anspruch 6, wobei Lactoferrin in der dritten Zusammensetzung mit einem Gehalt von mindestens 10 mg/100 kcal vorhanden ist.

8. Verfahren nach Anspruch 4, wobei Lactoferrin in der dritten Zusammensetzung mit einem Gehalt von 70 mg/100 kcal bis 220 mg/100 kcal vorhanden ist.

9. Verfahren nach Anspruch 1, wobei die Fett- oder Lipidquelle in der ersten Zusammensetzung mit einem Gehalt von ungefähr 3 g/100 kcal bis ungefähr 7 g/100 kcal vorhanden ist.

10. Verfahren nach Anspruch 1, wobei die Proteinquelle in der ersten Zusammensetzung mit einem Gehalt von ungefähr 1 g/100 kcal bis ungefähr 5 g/100 kcal vorhanden ist.

11. Zusammensetzung, umfassend eine Fett- oder Lipidquelle, eine Proteinquelle und ein hitzelabiles Milchprotein, hergestellt durch das Verfahren nach einem der Ansprüche 1, 6, 9 oder 10.

12. Zusammensetzung nach Anspruch 11, wobei das Verfahren nach Anspruch 1 ist und wobei das hitzelabile Milchprotein einer Sterilisation unterzogen wurde, wenn die zweite Zusammensetzung mit der ersten Zusammensetzung kombiniert ist.

13. Zusammensetzung nach Anspruch 11, wobei das Verfahren nach Anspruch 6 ist und wobei das hitzelabile Milchprotein Lactoferrin ist, bevorzugt ausgewählt aus der Gruppe bestehend aus nicht menschlichem Lactoferrin, menschlichem Lactoferrin, produziert durch einen genetisch modifizierten Organismus, und Kombinationen davon.

14. Zusammensetzung nach Anspruch 11, wobei das Verfahren nach Anspruch 1 ist und wobei die dritte Zusammensetzung eine Säuglingsnahrung ist.

## Revendications

1. Un procédé de préparation d'une composition comprenant une source de graisse ou de lipide, une source de protéines et une protéine de lait thermolabile, comprenant les étapes consistant à:
a) fournir une première composition comprenant une source de graisse ou de lipide et une source de protéine et soumettre la première composition à une température d'au moins environ 130°C;
b) fournir une deuxième composition comprenant une protéine de lait thermolabile; et
c) combiner la première composition avec la seconde composition comprenant une protéine de lait thermolabile pour former une troisième composition comprenant une source de graisse ou de lipide, une source de protéine et une protéine de lait thermolabile
procédé dans lequel la deuxième composition et la troisième composition ne sont pas soumises à une température supérieure à environ 80°C.

2. Le procédé selon la revendication 1, dans lequel la seconde composition est une solution aqueuse comprenant de l'eau qui a été traitée pour la transformation des aliments.

3. Le procédé selon la revendication 2, dans lequel l'eau a été traitée par osmose inverse.

4. Le procédé selon la revendication 1, dans lequel la seconde composition a été filtrée à travers un ou plusieurs filtres avant de combiner la deuxième composition avec la première composition.

5. Le procédé selon la revendication 1, comprenant en outre l'étape consistant à emballer la troisième composition de manière aseptique.

6. Le procédé selon la revendication 1, dans lequel la protéine de lait thermolabile est choisie dans le groupe constitué par la lactoferrine, la lactoferricine, le TGF-β, la lactopéroxydase, la lactadhérine, et des combinaisons de ceux-ci.

7. Le procédé selon la revendication 6, dans lequel la lactoferrine est présente dans la troisième composition en une proportion d'au moins 10 mg/100 kcal.

8. Le procédé selon la revendication 4 dans lequel la lactoferrin est présente dans la troisième composition en une proportion de 70 mg/100 kcal à 220 mg/100 kcal.

9. Le procédé selon la revendication 1, dans lequel la source de graisse ou de lipide est présente dans la première composition en une proportion d'environ 3 g/100 kcal à environ 7 g/100 kcal.

10. Le procédé selon la revendication 1, dans lequel la source de protéine est présente dans la première composition en une proportion d'environ 1 g/100 kcal à environ 5 g/100 kcal.

11. Une composition comprenant une source de graisse ou de lipide, une source de protéines et une protéine de lait thermolabile préparée par le procédé selon l'une quelconque des revendications 1, 6, 9 ou 10.

12. La composition selon la revendication 11, dans laquelle le procédé est selon la revendication 1, et dans laquelle la protéine de lait thermolabile a été soumise à une stérilisation lorsque la seconde composition est combinée avec la première composition.

13. La composition selon la revendication 11, dans laquelle le procédé est selon la revendication 6, et dans laquelle la protéine de lait thermolabile est la lactoferrine, de préférence choisie dans le groupe constitué par la lactoferrine non-humain, la lactoferrine humaine produite par un organisme génétiquement modifié, et des combinaisons de ceux-ci.

14. La composition selon la revendication 11, dans laquelle le procédé est selon la revendication 1, et dans laquelle la troisième composition est une formule pour nourrisson.
